# EUROPEAN PATENT APPLICATION

(11) **EP 4 400 841 A1**
(43) Date of publication of application: **17.07.2024**
(21) Application number: 22867392.7
(22) Date of filing: 08.09.2022
(51) Int. Cl.: G01N 33/68

(54) **METHOD FOR DETECTING ALZHEIMER'S DISEASE, AND DETECTION REAGENT**

(30) Priority: 08.09.2021 JP 2021146291
(71) Applicant: Tosoh Corporation, Yamaguchi 746-8501 (JP); Japanese Foundation For Cancer Research, Tokyo 135-8550 (JP); Igeta, Yukifusa, Tokyo 105-8470 (JP)
(72) Inventor: YUYAMA, Kohei, Sapporo-shi, Hokkaido 060-0808 (JP); UEDA, Koji, Tokyo 135-8550 (JP); IGETA, Yukifusa, Tokyo 105-8470 (JP)
(74) Representative: Novagraaf International SA
(86) International application number: PCT/JP2022/033637
(87) International publication number: WO 2023/038071

(57) **Abstract**

The present invention relates to providing of a method of simply detecting Alzheimer's disease (AD) and a method of simply stratifying AD pathological phases, and providing of a reagent that can be used for the methods. Based on the fact that measurement of a protein contained in extracellular vesicles enables detection of Alzheimer's disease and stratification of AD pathological phases, the present invention provides the method of simply detecting Alzheimer's disease (AD), the method of simply stratifying AD pathological phases, and the reagent that can be used for the methods.

## Description

### TECHNICAL FIELD

The present invention relates to a detection method and a detection reagent for Alzheimer's disease using proteins contained in extracellular (secreted) vesicles as measurement targets.

### BACKGROUND ART

Alzheimer's disease (AD) is the type of dementia with the largest number of patients. The pathogenesis of AD begins before the onset. It begins from the accumulation of amyloid β protein (Aβ), which is the early pathology, and gradually proceeds to neurofibrillary tangles of the Tau pathology and then to neurodegeneration, finally leading to the development of AD. At present, cerebrospinal fluid examination using the concentrations of Aβ and Tau proteins as indices, and PET imaging, are utilized as biomarkers for stratification of these pathological phases. However, the cerebrospinal fluid Aβ measurement is not covered by the insurance, and the collection of the cerebrospinal fluid is highly invasive, which is problematic. Furthermore, PET examination has many problems since,
example, it is costly, and is available only at a very limited number of facilities. Therefore, there is a demand for development of biomarkers that enable simple stratification of AD pathological phases for living brains, for the purposes of clinical and basic research uses aiming at treatment and prevention of AD.

### SUMMARY OF THE INVENTION

### PROBLEMS TO BE SOLVED BY THE INVENTION

An object of the present invention is to provide a method of simply detecting Alzheimer's disease (AD) and a method of simply stratifying AD pathological phases for living brains even before the onset of AD. Another object is to provide a reagent that can be used for the methods.

### MEANS FOR SOLVING THE PROBLEMS

As a result of intensive study, the present inventors discovered that a specific group of proteins in extracellular vesicles exhibits variation across different pathological phases of AD based on indicative abnormal proteins such as Aβ and Tau. The inventors then identified the proteins within extracellular vesicles that demonstrated variations among these pathological phases. This leads the inventors to inferred that the measurement of proteins contained in extracellular vesicles could facilitate the detection of Alzheimer's disease and stratification of AD pathological phases, thereby culminating in the completion of the present invention.

More specifically, the present invention encompasses the following aspects.
[1] A method of detecting Alzheimer's disease, comprising the step of:
   measuring the quantity of a protein present in extracellular vesicles in a sample collected from a specimen.
[2] The method according to [1], wherein the amount of the protein contained in extracellular vesicles in an Alzheimer's disease specimen is different from an amount of the protein contained in extracellular vesicles in a normal specimen.
[3] The method according to [2], wherein the difference is by a factor of not less than 2 or not more than 0.5.
[4] The method according to any one of [1] to [3], comprising the step of:
   collecting an extracellular vesicle fraction from the sample collected from the specimen, and measuring the protein contained in extracellular vesicles in the extracellular vesicle fraction.
[5] The method according to any one of [1] to [4], wherein the protein contained in the extracellular vesicles is one or more proteins selected from the group consisting of: ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA 2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B 10, Nodal modulator 1, Nodal modulator 2, Nodal modulator 3, phosphoribosyltransferase domain-containing protein 1, protein furry homolog-like, myosin-10, helicase senataxin, protein FAM227B, serpin B4, cholinesterase, pericentrin, protein unc-13 homolog C, 26S proteasome (proteosome) non-ATPase regulatory subunit 1, neurexophilin 1, DNA-binding protein RFX5, pregnancy-specific beta-1-glycoprotein 8, amyloid-beta A4 precursor protein-binding family B member 2, kallikrein-11, cadherin-11, sodium channel protein type 4 subunit alpha, and transmembrane protein 62.
[6] The method according to any one of [1] to [5], wherein the protein contained in the extracellular vesicles includes cathepsin B.
[7] A method of detecting an amyloid accumulation phase of Alzheimer's disease, the method comprising the step of:
   measuring a protein contained in extracellular vesicles in a sample collected from a specimen.
[8] The method according to [7], wherein the protein contained in the extracellular vesicles is one or more proteins selected from the group consisting of: ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA 2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B10, Nodal modulator 1, Nodal modulator 2, Nodal modulator 3, phosphoribosyltransferase domain-containing protein 1, protein furry homolog-like, myosin-10, helicase senataxin, protein FAM227B, and serpin B4.
[9] The method according to [7] or [8], wherein the protein contained in the extracellular vesicles includes cathepsin B.
[10] A method of detecting a Tau-pathogenesis phase and/or a neurodegenerative phase of Alzheimer's disease, the method comprising the step of:
   measuring a protein contained in extracellular vesicles in a sample collected from a specimen.
[11] The method according to [10], wherein the protein contained in the extracellular vesicles is one or more proteins selected from the group consisting of: cholinesterase, pericentrin, protein unc-13 homolog C, 26S proteasome non-ATPase regulatory subunit 1, alpha-(1,3)-fucosyltransferase 10, neurexophilin 1, DNA-binding protein RFX5, pregnancy-specific beta-1-glycoprotein 8, amyloid-beta A4 precursor protein-binding family B member 2, kallikrein-11, cadherin-11, beta-galactoside alpha-2,6-sialyltransferase 1, sodium channel protein type 4 subunit alpha, protein FAM227B, and transmembrane protein 62.
[12] The method according to [10] or [11], wherein the protein contained in the extracellular vesicles includes cathepsin B.
[13] The method according to any one of [1] to [12], wherein the sample is cerebrospinal fluid or plasma.
[14] A diagnostic kit for Alzheimer's disease, comprising a reagent for measuring a protein contained in extracellular vesicles.

### ADVANTAGEOUS EFFECTS OF THE INVENTION

By the present invention, a method of simply detecting Alzheimer's disease (AD), a method of simply stratifying AD pathological phases for living brains even before the onset of AD, and a reagent that can be used for these methods, are provided.

### BRIEF DESCRIPTION OF THE DRAWINGS

Fig. 1 is a graph showing the cathepsin B concentration in an extracellular vesicle fraction of cerebrospinal fluid in subjects in each ATN group. ***: p < 0.001.
Fig. 2A is a graph showing the cathepsin B concentration in an extracellular vesicle fraction of cerebrospinal fluid, as observed for cases where the amyloid pathology was present or absent (subjects in the A+ group or A- group). ***: p < 0.001.
Fig. 2B is a graph showing correlation between the cerebrospinal fluid Aβ42 concentration, and the cathepsin B concentration in an extracellular vesicle fraction of cerebrospinal fluid, in each subject. r represents the correlation coefficient.
Fig. 3A is a graph showing the cathepsin B concentration in an extracellular vesicle fraction of plasma as observed for cases where the amyloid pathology was present or absent (subjects in the A+ group or A- group). ***: p < 0.001.
Fig. 3B is a graph showing correlation between the cerebrospinal fluid Aβ42 concentration, and the cathepsin B concentration in an extracellular vesicle fraction of plasma, in each subject. r represents the correlation coefficient.

### EMBODIMENTS FOR CARRYING OUT THE INVENTION

A first aspect of the present invention is a method of detecting Alzheimer's disease, comprising the step of:
measuring the amount of a protein contained in extracellular vesicles in a sample collected from a specimen.

The specimen is not limited as long as it is an animal which potentially develops Alzheimer's disease. The specimen is preferably a mammal, more preferably a human.

The detection method according to the present invention is usually carried out in vitro.

Examples of the sample include body fluids, cells, and tissues collected from specimens. Examples of the body fluids include cerebrospinal fluid, blood, lymph, and tissue fluid. Examples of the blood include plasma and blood cell components. The sample is preferably cerebrospinal fluid or plasma, more preferably cerebrospinal fluid.

The collection of the sample from the specimen may be carried out by an appropriate method including a known method, depending on the sample to be collected and the specimen from which the sample is to be collected. For example, cerebrospinal fluid can be collected by performing lumbar puncture and directly collecting drops, and plasma can be collected by subjecting collected blood to centrifugation. However, the collection method is not limited to these. The collected sample may be subjected, when necessary, to centrifugation, cryopreservation, and/or the like.

The extracellular vesicles refer to vesicles released by cells. Typically, an extracellular vesicle carries the cell membrane components from its source cell on its surface and encapsulates substances from the source cell within.

The protein found in extracellular vesicles can exist within the vesicles, on the surfaces of the vesicles, or as a transmembrane protein of the vesicles. The protein selection is not restricted as long as it is collected alongside the extracellular vesicles. Preferably, the protein within extracellular vesicles is a component of an extracellular vesicle fraction.

The measurement of the protein contained in extracellular vesicles may be carried out for an entire sample, or may be carried out for an extracellular vesicle fraction of a sample. Thus, the present invention may comprise the step of collecting an extracellular vesicle fraction from a sample collected from a specimen.

The extracellular vesicle fraction is a fraction containing a large amount of extracellular vesicles in a sample. The fraction can be obtained by fractionation utilizing, for example, ultracentrifugal precipitation, density gradient ultracentrifugal fractionation, size exclusion chromatography, an antibody against a surface antigen, or a method of capturing a lipid that is specifically present on the surfaces of extracellular vesicles.

The extracellular vesicle fraction can be collected by ultracentrifugation, column purification, or the like. The collection can also be carried out by using a commercially available product. Examples of such a product include, but are not limited to, EV-Second L70 (manufactured by GL Sciences Inc.), which is a size exclusion chromatography column.

For the measurement of the protein contained in extracellular vesicles, a known protein measurement method may be used. The measurement may be preferably carried out by mass spectrometry using a liquid chromatograph mass spectrometer (LC-MS), or by an immunoassay using an antibody against the protein contained in extracellular vesicles. Examples of the immunoassay include a method in which quantitative detection is carried out by an enzyme immunoassay, and a method in which measurement is carried out by fluorescence immunoassay, chemiluminescent immunoassay, or the like. The enzyme immunoassay, among the labeled immunoassays, is a detection method using an enzyme as a labeling substance. Examples of the enzyme immunoassay include the ELISA method.

Examples of the ELISA method include the direct method, indirect method, and sandwich method. Any of these methods may be used. The direct method of the ELISA method may be a method well known in the art in which a labeled antibody is bound to a protein in a biological sample immobilized on a solid phase, and then the labeling substance is detected. The indirect method of the ELISA method may be a method well known in the art in which a primary antibody is bound to a protein in a biological sample immobilized on a solid phase, to form an immune complex on the surface of the solid phase, and then a labeled secondary antibody that recognizes the primary antibody is used, followed by detecting the labeling substance. The sandwich method of the ELISA method may be a method well known in the art in which an immobilized antibody (primary antibody) immobilized on a solid phase is allowed to capture a protein, and then a labeled secondary antibody is bound to the captured protein, to form, on the surface of the solid phase, an immune complex containing the two kinds of bound antibodies, followed by detecting the labeling substance.

LC-MS is an apparatus comprising the combination of a high-performance liquid chromatograph (HPLC) and a mass spectrometer (MS). Measurement by mass spectrometry using LC-MS allows identification and quantification of a protein. Examples of the MS to be used for the LC-MS include, but are not limited to, Orbitrap Fusion Lumos (manufactured by Thermo Scientific).

The protein may be identified by a method suitable for each protein measurement method, and the method of the identification is not limited. For example, in cases of measurement by mass spectrometry using LC-MS, a protein database may be searched with product ion spectrum data obtained by the analysis using the LC-MS. Examples of the protein database include the public protein database SwissProt.

The protein may be quantified by a method suitable for each protein measurement method, and the method of the quantification is not limited. For example, in cases of measurement by mass spectrometry using LC-MS, calculation may be performed using a peak area based on precursor ion spectrum data obtained by the analysis using the LC-MS. The identification and the quantification of the protein may be carried out using software. Examples of such software include, but are not limited to, Proteome Discoverer 2.4 software (manufactured by Thermo Fischer Scientific).

From the sample to be used for the protein measurement, the protein may be purified. Examples of the protein purification method include protein purification methods using polyacrylamide electrophoresis (SDS-PAGE). However, the method is not limited thereto, and may be appropriately selected depending on the sample used, the protein measurement method, or the like. The sample may be used for the protein measurement without purification of the protein. The sample may be diluted, when appropriate.

Large-scale analysis of proteins contained in extracellular vesicles is called extracellular vesicle proteomics. The extracellular vesicle proteomics may be comprehensive analysis of the proteins present in extracellular vesicles. In one aspect of the extracellular vesicle proteomics, for example, the concentrations of protein molecular species contained in extracellular vesicles are comprehensively analyzed by mass spectrometry using LC-MS.

The extracellular vesicle proteomics can be carried out, for example, by subjecting an extracellular vesicle fraction of a sample to mass spectrometry using LC-MS, and identifying and quantifying proteins based on the analytical data.

Data obtained by the proteomics analysis, containing a list of quantitative data of identified proteins, are also referred to as a protein profile. Therefore, the data obtained by the extracellular vesicle proteomics, containing a list of quantitative data of identified extracellular vesicle proteins, are also referred to as a profile of proteins contained in extracellular vesicles.

Extracellular secreted vesicle proteomics shows a difference between a profile of proteins contained in extracellular vesicles of a normal specimen and a profile of proteins contained in extracellular vesicles of an Alzheimer's disease specimen. Therefore, the protein contained in extracellular vesicles measured in the present invention preferably shows a difference in its amount between an Alzheimer's disease specimen and a normal specimen.

When the amount of the protein contained in extracellular vesicles of a specimen shows not less than a certain amount of change in the measured value during a predetermined period, is more than or less than the amount of the protein contained in extracellular vesicles of a normal specimen, or is more than or less than a set threshold, the above specimen is detected to have Alzheimer's disease or a risk of developing Alzheimer's disease. In the present description, "risk of developing Alzheimer's disease" means that a specimen has not developed Alzheimer's disease at the time of the measurement, but is highly likely to develop the disease in the near future.

In cases of a protein contained in extracellular vesicles which is at a higher level in a profile of proteins contained in extracellular vesicles of an Alzheimer's disease specimen than in a profile of proteins contained in extracellular vesicles of a normal specimen, when the amount of the protein contained in extracellular vesicles of a specimen shows not less than a certain level of increase in the measured value during a predetermined period, is at a higher level than the amount of the protein contained in extracellular vesicles of a normal specimen, or shows a level of not less than a set threshold, the above specimen is preferably detected to have Alzheimer's disease or a risk of developing Alzheimer's disease.

Specific examples of the detection criterion may include, for example, the fact that the protein shows an amount which is not less than 1.05 times, not less than 1.5 times, not less than 2.0 times, not less than 3.0 times, or not less than 5.0 times the amount of the protein found in a profile of proteins contained in extracellular vesicles of a normal specimen. The detection criterion is preferably the fact that the protein shows an amount which is not less than 2.0 times the amount of the protein found in a profile of proteins contained in extracellular vesicles of a normal specimen.

In cases of a protein contained in extracellular vesicles which is at a lower level in a profile of proteins contained in extracellular vesicles of an Alzheimer's disease specimen than in a profile of proteins contained in extracellular vesicles of a normal specimen, when the amount of the protein contained in extracellular vesicles of a specimen shows not less than a certain level of decrease in the measured value during a predetermined period, is at a lower level than the amount of the protein contained in extracellular vesicles of a normal specimen, or shows a level of not more than a set threshold, the above specimen is preferably detected to have Alzheimer's disease or a risk of developing Alzheimer's disease.

Specific examples of the detection criterion may include, for example, the fact that the protein shows an amount which is not more than 0.95 times, not more than 0.75 times, not more than 0.5 times, not more than 0.3 times, or not more than 0.2 times the amount of the protein found in a profile of proteins contained in extracellular vesicles of a normal specimen. The detection criterion is preferably the fact that the protein shows an amount which is not more than 0.5 times the amount of the protein found in a profile of proteins contained in extracellular vesicles of a normal specimen.

Alzheimer's disease is a dementia which exhibits symptoms such as disorientation, impaired delayed recall, impaired visuospatial ability, and impaired executive function, and whose causative gene or causative protein such as amyloid β has been identified and diagnosed. A dementia diagnosed only by clinical symptoms, without diagnosis by the gene or protein, is referred to as Alzheimer's type of dementia. A prodromal state of Alzheimer's disease (Alzheimer's type of dementia) is amnestic mild cognitive impairment (amnestic MCI: MCI).

Alzheimer's disease is typically characterized by accumulation of amyloid β outside neurons, in the cerebral cortex, or in cerebral blood vessels; accumulation of phosphorylated tau in neurons; and neurodegenerative pathologies.

More typically, Alzheimer's disease first exhibits accumulation of amyloid β, and then accumulation of phosphorylated tau occurs, followed by sequential progression to neurofibrillary tangles, neurodegeneration, cerebral atrophy, and then cognitive impairment. The phases of Alzheimer's disease, represented by these, are referred to as Alzheimer's disease pathological phases (AD pathological phases).

Among the AD pathological phases, the phase that exhibits the accumulation of amyloid β is also referred to as amyloid accumulation phase. The phase that exhibits the accumulation of phosphorylated tau is also referred to as tau pathogenesis phase. The phase that exhibits the neurodegeneration is also referred to as neurodegenerative phase.

Examples of a conventional method of biological stratification of the AD pathological phases include the ATN classification method, in which specimens are divided based on the presence or absence of the amyloid accumulation, tau pathology, and neurodegeneration, and in which the phases are classified mainly based on abnormal proteins showing variation that allows the classification for living brains. Thus, the ATN classification method stratifies the AD pathological phases into the amyloid accumulation phase, the tau pathogenesis phase, and the neurodegenerative phase.

In the ATN classification method, amyloid β, phosphorylated tau, and total tau are measured for a specimen of a living patient. The measurement of amyloid β, phosphorylated tau, and total tau can be carried out by a known method such as the ELISA method. Based on the measured values, classification is performed for amyloid β (A), phosphorylated tau (T), and total tau (N). The classification may be, for example, based on positivity (+) or negativity (-) determined by comparing the measured value for each of A, T, and N with a cut-off value. More specifically, for example, an amyloid β value lower than its cut-off value may be rated as A+; a phosphorylated tau value higher than its cut-off value may be rated as T+; a total tau value higher than its cut-off value may be rated as N+; and, for each of A, T, and N, values other than those described above may be rated as negative (-). In the ATN classification, for example, A-T-N-is classified as the normal state; A+T-N- is classified as the amyloid accumulation phase; and A+T+N+ is classified as the tau pathogenesis/ neurodegenerative phase. The cut-off values for the ATN classification may be, but do not necessarily need to be, set in accordance with the result of ROC analysis between a normal + MCI group and an AD group based on clinical diagnosis.

By combining a conventional method of stratification of the AD pathology phases with the extracellular vesicle proteomics, a profile of proteins contained in extracellular vesicles in each AD pathological phase can be elucidated. For example, for specimens classified by the ATN classification method, profiles of proteins contained in extracellular vesicles of A-T-N-, A+T-N-, or A+T+N+ specimens reflect profiles of proteins contained in extracellular vesicles in the normal state, the amyloid accumulation phase, or the tau pathogenesis/neurodegenerative phase, respectively.

Specimens from living patients corresponding to the different AD pathological phases of Alzheimer's disease exhibit different protein profiles in the extracellular vesicle proteomics.

Thus, when the amount of a protein contained in extracellular vesicles of a specimen shows not less than a certain amount of change in the measured value during a predetermined period, is more than or less than the amount of the protein contained in extracellular vesicles of a normal specimen, or is more than or less than a set threshold, the above specimen may be detected to be in a particular AD pathological phase.

A protein is regarded as an amyloid accumulation phase marker protein in cases where the protein exhibits a difference in the measured value when a profile of proteins contained in extracellular vesicles of a specimen in the amyloid accumulation phase of Alzheimer's disease is compared to a profile of proteins contained in extracellular vesicles of a normal specimen. The amyloid accumulation phase marker protein is preferably a protein that exhibits a difference in the measured value by a factor of not less than 2 or not more than 0.5 when a profile of proteins contained in extracellular vesicles of a specimen in the amyloid accumulation phase of Alzheimer's disease is compared to a profile of proteins contained in extracellular vesicles of a normal specimen. The amyloid accumulation phase marker protein is more preferably a protein(s) selected from the group consisting of ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B 10 (melanoma-associated antigen B 10), Nodal modulator 1, Nodal modulator 2, Nodal modulator 3, phosphoribosyltransferase domain-containing protein 1, protein furry homolog-like, myosin-10, helicase senataxin, protein FAM227B, and serpin B4.

The amyloid accumulation phase marker protein more preferably includes cathepsin B. Cathepsin B may serve both as an amyloid accumulation phase marker protein and as a tau pathogenesis/neurodegenerative phase marker protein at the same time.

When the amount of the amyloid accumulation phase marker protein in a specimen shows not less than a certain amount of change in the measured value during a predetermined period, is more than or less than the amount of the amyloid accumulation phase marker protein in a normal specimen, or is more than or less than a set threshold, the above specimen may be detected to be in the amyloid accumulation phase.

More specifically, in cases of an amyloid accumulation phase marker protein which is at a higher level in a profile of proteins contained in extracellular vesicles of a specimen in the amyloid accumulation phase of Alzheimer's disease than in a profile of proteins contained in extracellular vesicles of a normal specimen, when the amount of the amyloid accumulation phase marker protein in a specimen shows not less than a certain level of increase in the measured value during a predetermined period, is at a higher level than the amount of the amyloid accumulation phase marker protein in a normal specimen, or shows a level of not less than a set threshold, the above specimen is preferably detected to be in the amyloid accumulation phase of Alzheimer's disease, or to have Alzheimer's disease, MCI, or a risk of developing Alzheimer's disease.

More specific examples of the detection criterion may include, for example, the fact that the protein shows an amount which is not less than 1.05 times, not less than 1.5 times, not less than 2.0 times, not less than 3.0 times, or not less than 5.0 times the amount of the protein found in a profile of proteins contained in extracellular vesicles of a normal specimen. The detection criterion is preferably the fact that the protein shows an amount which is not less than 2.0 times the amount of the protein found in a profile of proteins contained in extracellular vesicles of a normal specimen.

Examples of the above-described amyloid accumulation phase marker protein which is at a higher level in a profile of proteins contained in extracellular vesicles of a specimen in the amyloid accumulation phase of Alzheimer's disease than in a profile of proteins contained in extracellular vesicles of a normal specimen include, but are not limited to, cathepsin B.

In cases of an amyloid accumulation phase marker protein which is at a lower level in a profile of proteins contained in extracellular vesicles of a specimen in the amyloid accumulation phase of Alzheimer's disease than in a profile of proteins contained in extracellular vesicles of a normal specimen, when the amount of the amyloid accumulation phase marker protein in a specimen shows not less than a certain level of decrease in the measured value during a predetermined period, is at a lower level than the amount of the amyloid accumulation phase marker protein in a normal specimen, or shows a level of not more than a set threshold, the above specimen is preferably detected to be in the amyloid accumulation phase of Alzheimer's disease, or to have Alzheimer's disease, MCI, or a risk of developing Alzheimer's disease.

More specific examples of the detection criterion may include, for example, the fact that the protein shows an amount which is not more than 0.95 times, not more than 0.75 times, not more than 0.5 times, not more than 0.3 times, or not more than 0.2 times the amount of the protein found in a profile of proteins contained in extracellular vesicles of a normal specimen. The detection criterion is preferably the fact that the protein shows an amount which is not more than 0.5 times the amount of the protein found in a profile of proteins contained in extracellular vesicles of a normal specimen.

A protein is regarded as a tau pathogenesis/neurodegenerative phase marker protein in cases where the protein exhibits a difference in the measured value when a profile of proteins contained in extracellular vesicles of a specimen in the tau pathogenesis/neurodegenerative phase of Alzheimer's disease is compared to a profile of proteins contained in extracellular vesicles of a normal specimen. The tau pathogenesis/neurodegenerative phase marker protein is preferably a protein that exhibits a difference in the measured value by a factor of not less than 2 or not more than 0.5 when a profile of proteins contained in extracellular vesicles of a specimen in the tau pathogenesis/neurodegenerative phase of Alzheimer's disease is compared to a profile of proteins contained in extracellular vesicles of a normal specimen. The tau pathogenesis/neurodegenerative phase marker protein is more preferably a protein(s) selected from the group consisting of cholinesterase, pericentrin, protein unc-13 homolog C, 26S proteasome non-ATPase regulatory subunit 1, alpha-(1,3)-fucosyltransferase 10, neurexophilin-1, DNA-binding protein RFX5, pregnancy-specific beta-1-glycoprotein 8, amyloid-beta A4 precursor protein-binding family B member 2, kallikrein-11, cadherin-11,beta-galactoside alpha-2,6-sialyltransferase 1, sodium channel protein type 4 subunit alpha, protein FAM227B, and transmembrane protein 62.

The tau pathogenesis/neurodegenerative phase marker protein may include cathepsin B. In this case, the tau pathogenesis/neurodegenerative phase marker protein may be cathepsin B alone, or may be the combination of cathepsin B and another tau pathogenesis/neurodegenerative phase marker protein. Cathepsin B may serve both as a tau pathogenesis/neurodegenerative phase marker protein and as an amyloid accumulation phase marker protein at the same time.

When the amount of the tau pathogenesis/neurodegenerative phase marker protein shows not less than a certain amount of change in the measured value during a predetermined period, is more than or less than the amount of the tau pathogenesis/neurodegenerative phase marker protein in a normal specimen, or is more than or less than a set threshold, the above specimen may be detected to be in the tau pathogenesis/neurodegenerative phase.

More specifically, in cases of a tau pathogenesis/neurodegenerative phase marker protein which is at a higher level in a profile of proteins contained in extracellular vesicles of a specimen in the tau pathogenesis/neurodegenerative phase of Alzheimer's disease than in a profile of proteins contained in extracellular vesicles of a normal specimen, when the amount of the tau pathogenesis/neurodegenerative phase marker protein in a specimen shows a certain level of increase in the measured value during a predetermined period, is at a higher level than the amount of the tau pathogenesis/neurodegenerative phase marker protein in a normal specimen, or shows a level of not less than a set threshold, the above specimen is preferably detected to be in the tau pathogenesis/neurodegenerative phase of Alzheimer's disease, or to have Alzheimer's disease.

Specific examples of the detection criterion may include, for example, the fact that the protein shows an amount which is not less than 1.05 times, not less than 1.5 times, not less than 2.0 times, not less than 3.0 times, or not less than 5.0 times the amount of the protein found in a profile of proteins contained in extracellular vesicles of a normal specimen. The detection criterion is preferably the fact that the protein shows an amount which is not less than 2.0 times the amount of the protein found in a profile of proteins contained in extracellular vesicles of a normal specimen.

Examples of the above-described amyloid accumulation phase marker protein which is at a higher level in a profile of proteins contained in extracellular vesicles of a specimen in the tau pathogenesis/neurodegenerative phase of Alzheimer's disease than in a profile of proteins contained in extracellular vesicles of a normal specimen include, but are not limited to, cathepsin B.

In cases of a tau pathogenesis/neurodegenerative phase marker protein which is at a lower level in a profile of proteins contained in extracellular vesicles of a specimen in the tau pathogenesis/neurodegenerative phase of Alzheimer's disease than in a profile of proteins contained in extracellular vesicles of a normal specimen, when the amount of the tau pathogenesis/neurodegenerative phase marker protein in a specimen shows a certain level of decrease in the measured value during a predetermined period, is at a lower level than the amount of the tau pathogenesis/neurodegenerative phase marker protein in a normal specimen, or shows a level of not more than a set threshold, the above specimen is preferably detected to be in the tau pathogenesis/neurodegenerative phase of Alzheimer's disease, or to have Alzheimer's disease.

Specific examples of the detection criterion may include, for example, the fact that the protein shows an amount which is not more than 0.95 times, not more than 0.75 times, not more than 0.5 times, not more than 0.3 times, or not more than 0.2 times the amount of the protein found in a profile of proteins contained in extracellular vesicles of a normal specimen. The detection criterion is preferably the fact that the protein shows an amount which is not more than 0.5 times the amount of the protein found in a profile of proteins contained in extracellular vesicles of a normal specimen.

The method according to the first aspect of the present invention includes the process until the stage of detecting Alzheimer's disease, but does not include the act of making a final judgment on the diagnosis of Alzheimer's disease. A physician may refer to the detection result by the method of the present invention and the like to perform diagnosis of Alzheimer's disease, and to determine the course of treatment of the disease.

A second aspect of the present invention is a diagnostic kit for Alzheimer's disease, comprising a reagent for measuring a protein contained in extracellular vesicles.

To the measurement of the protein contained in extracellular vesicles using the reagent for measuring a protein contained in extracellular vesicles, the above description can be applied.

The reagent for measuring a protein contained in extracellular vesicles is not limited as long as it is capable of detecting and/or quantifying the protein contained in extracellular vesicles. The reagent is preferably capable of quantifying the protein contained in extracellular vesicles.

The kit may comprise a reagent, a tool, or the like to be used as means to isolate a sample from a specimen. This means may include means to isolate cerebrospinal fluid or blood from the specimen, and may also include means to isolate plasma from blood. The means to isolate a sample may also include means to collect an extracellular vesicle fraction.

The diagnostic kit of the present invention can be produced using a reagent for measuring a protein contained in extracellular vesicles. Thus, another aspect of the present invention includes use of a reagent for measuring a protein contained in extracellular vesicles, for the production of a diagnostic kit for Alzheimer's disease.

To the reagent for measuring a protein contained in extracellular vesicles, the above description can be applied. Examples of the reagent include, but are not limited to, antibodies that recognize a protein contained in extracellular vesicles. The reagent for measuring a protein contained in extracellular vesicles preferably comprises, but does not necessarily need to comprise, an antibody that recognizes an amyloid accumulation phase marker protein or a tau pathogenesis/neurodegenerative phase marker protein. The reagent more preferably comprises an antibody that recognizes cathepsin B.

A third aspect of the present invention is a method of treating Alzheimer's disease in a patient, the method comprising the steps of:
(i) identifying a patient based on a measured value of a marker, which measured value is above or below a reference value set in advance; and
(ii) treating the identified patient.

In the identification in Step (i), the measurement of the marker may be carried out using an antibody that specifically recognizes the marker, or using mass spectrometry.

"Above or below a reference value" means that the measured value is higher or lower than a reference value, and the reference value is preferably set depending on the marker.

The marker is preferably a protein contained in extracellular vesicles, more preferably an amyloid accumulation phase marker protein or a tau pathogenesis/neurodegenerative phase marker protein, still more preferably cathepsin B.

To the method of treating Alzheimer's disease of the present invention, the method of detecting Alzheimer's disease of the present invention may be applied. Thus, a method according to the third aspect of the present invention is provided by the above-described first aspect of the present invention. More specifically, for example, in the identification in Step (i), the patient may be identified based on detection of Alzheimer's disease by a method according to the first aspect of the present invention; the patient may be identified based on detection of the amyloid accumulation phase of Alzheimer's disease by a method according to the first aspect of the present invention; or the patient may be identified based on detection of the tau pathogenesis phase and/or neurodegenerative phase of Alzheimer's disease by a method according to the first aspect of the present invention.

The treatment is not limited, and may be selected appropriately according to judgement by a physician.

### EXAMPLES

### (Example 1)

### Spinal Fluid Extracellular Secreted Vesicle Proteomics

### Details of Subjects

For cerebrospinal fluid extracellular vesicle proteomics, specimens from the following subjects (n = 16) were used (Table 1).

**Table 1**

| No | ATN classification | Clinical diagnosis classification | Number of specimen |
|---|---|---|---|
| 1 | A+T+N+ | AD | 4 |
| 2 | A+T-N- | AD/MCI/ Normal | 8 |
| 3 | A-T-N- | AD | 4 |
| | | Total | 16 |

### Collection of Spinal Fluid

Patients satisfying the following conditions were subjected to cerebrospinal fluid examination.
- The patients were 40 to 80 years old, and taking neither an antiplatelet drug nor anticoagulant.
- The patients had finished dinner by 21:00, and slept for at least 6 hours without taking alcohol on the day before the collection.
- The patients were allowed to drink water, not allowed to drink tea or coffee, and not allowed to have breakfast, to enable collection of cerebrospinal fluid between 9:00 AM and 10:00 AM at fasting.

### Method of Collection of Spinal Fluid

For a patient in the left lateral decubitus position, lumbar puncture is performed between the third and fourth lumbar vertebrae or between the fourth and fifth lumbar vertebrae.

For the puncture, a 22G top spinal needle with stainless-steel needle tubing and a polypropylene needle base is used. Cerebrospinal fluid specimen that drops from the needle hub of the spinal needle is directly collected without using any of a polystyrene spinal needle, a three-way stopcock, and an extension tube, which are usually used.

First, about 500 µl of cerebrospinal fluid is directly collected dropwise into a sterile 15-cc polypropylene spitz tube. After replacing the container with a polypropylene microtube (1.5 ml), 500 µl of cerebrospinal fluid is directly collected dropwise from the needle hub into the microtube. Thereafter, cerebrospinal fluid is again directly collected dropwise into the same sterile 15-cc spitz tube, and then cerebrospinal fluid is collected dropwise into another 1.5-ml polypropylene microtube in an amount of 500 µl/tube. These steps are then repeated. The operation is continued until cerebrospinal fluid is collected into one sterile 15-cc spitz tube (about 4 to 5 cc of cerebrospinal fluid) and a total of 10 to 12 microtubes (each containing 500 µl of cerebrospinal fluid).

When gross hemorrhage is found, the operation is stopped.

The cerebrospinal fluid in each microtube, in an amount of 500 µl, is cryopreserved as it is at -80°C within 1 hour after the beginning of the collection (specimen for analysis of extracellular vesicles).

The cerebrospinal fluid specimen collected in the propylene microtube is centrifuged at 3500 rpm (2200 × g) for 7 minutes, and then the supernatant is aliquoted in 500-µl volumes into polypropylene microtubes (1.5 ml) using a polypropylene dispensing tip, followed by immediately cryopreserving the specimens at -80°C (specimens for ELISA for Aβ40, Aβ42, pTau, and tTau).

All of the above steps are performed by the same operator during the same period using tools made of the same materials.

### ATN Classification

ELISA was carried out to measure Aβ (Aβ1-42), phosphorylated tau (p-Tau181), and total tau (t-Tau) in the cerebrospinal fluid specimens collected from the subjects, and the subjects were divided based on the measured values according to the ATN classification (Aβ (A), phosphorylated tau (T), and neurodegeneration (N)). Cut-off values were set by performing ROC analysis between a normal + MCI group and an AD group, based on clinical diagnoses of all subjects. In the present Example, the cut-off values were as follows: Aβ: 680 pg/mL, phosphorylated tau: 76.5 pg/mL, and total tau: 598 pg/mL. Even by recalculation using the cut-off values set in Example 2 below (Aβ: 747.2 pg/mL, phosphorylated tau: 58.4 pg/mL, and total tau: 756.6 pg/mL), the ATN classification of the subjects in the present Example did not change from that described in Table 1.

### Preparation of Extracellular Secreted Vesicle Fraction

From 0.5 mL of the cerebrospinal fluid, an extracellular vesicle fraction was prepared. The preparation of the extracellular vesicle fraction was carried out using an extracellular vesicle purification column (EV-Second L70, manufactured by GL Sciences Inc.).

### Proteomics Analysis (LC-MS)

Comprehensive identification and quantification of proteins in the extracellular vesicle fraction were carried out by LC-MS analysis. More specifically, the analysis was carried out as follows.

### (1) Sample Preparation

The extracellular vesicle fraction was dissolved in 30 µl of Laemmli's sample buffer, and then reduced with 10 mM TCEP at 100°C for 10 minutes, followed by alkylation with 50 mM iodoacetamide. SDS-PAGE was carried out, and protein bands were excised from the gel, followed by performing digestion with trypsin/Lys-C-Mix. The resulting digest was subjected to LC-MS analysis.

### (2) LC-MS Analysis

The analysis was carried out using Orbitrap Fusion Lumos mass spectrometer (manufactured by Thermo Scientific) equipped with the Ultimate 3000 RSLC nano-flow HPLC system (manufactured by DIONEX Corporation, USA).

Using Proteome Discoverer 2.4 software (manufactured by Thermo Scientific), identification of proteins was attempted by searching the SwissProt human protein database.

### (3) Label-Free Quantitative Analysis

The LC-MS dataset was subjected to label-free quantitative analysis using Proteome Discoverer 2.4 software (manufactured by Thermo Scientific), to perform relative quantification of all detected proteins.

### (4) Results

From the cerebrospinal fluid extracellular vesicle fraction, quantitative data were obtained for 1758 proteins. Each protein was judged to have some effectiveness as a biomarker for the detection of the amyloid accumulation phase in cases where the ratio of the average amount of the protein in the extracellular vesicle fraction of Group 2 (A+T-N- group) to the average amount of the protein in the extracellular vesicle fraction of Group 3 (A-T-N- group) was not less than 2 or not more than 0.5, or where the expression of the protein was found only in one of Group 1 and Group 2. As a result, the following molecular species were selected (Table 2): ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B 10 (melanoma-associated antigen B 10), Nodal modulator 1, Nodal modulator 2, Nodal modulator 3, phosphoribosyltransferase domain-containing protein 1, protein furry homolog-like, myosin-10, helicase senataxin, protein FAM227B, and serpin B4. In addition, these molecular species may be useful as potential targets of therapeutic/prophylactic agents.

**Table2 A + T - N - / A - T - N -**

| **Accession No.** | **Protein name** | **Abundances (A+)** | **Abundances (A-)** | **Ratio (A+/A-)** | **p value (t test)** |
|---|---|---|---|---|---|
| O14638 | Ectonucleotide pyrophcsphatase/phosphodiesterase family member 3 | 642285 | 191615 | 3.35 | 0.0113 |
| Q6P4F1 | Alpha-(1,3)-fucosyltransferase 10 | 351706 | 109584 | 3.21 | 0.0138 |
| Q96B86 | Repulsive guidance molecule A | 1672251 | 575450 | 2.91 | 0.0374 |
| P31321 | cAMP-dependent protein kinase type I-beta regulatory subunit | 595422 | 220472 | 2.70 | 0.0196 |
| P51530 | DNA replication ATP-dependent helicase/nuclease DNA2 | 369963 | 158361 | 2.34 | 0.0415 |
| Q9UQ52 | Contactin-6 | 3301752 | 7637923 | 0.43 | 0.0147 |
| P62805 | Histone H4 | 12699883 | 32682495 | 0.39 | 0.0308 |
| Q16394 | Exostosin-1 | 168450 | 436580 | 0.39 | 0.0188 |
| Q5H9F3 | BCL-6 corepressor-like protein 1 | 217158 | 718971 | 0.30 | 0.0087 |
| P0DOX6 | Immunoglobulin mu heavy chain | 2622293 | 11795177.7 | 0.22 | 0.0179 |
| Q8N1W1 | Rho guanine nucleotide exchange factor 28 | 1459310 | | | |
| P07858 | Cathepsin B | 595868 | | | |
| A6NCN2 | Putative keratin-87 protein | 2810219 | | | |
| Q93062 | RNA-birding protein with multiple splicing | 885404 | | | |
| P29508 | Serpin B3 | 879184 | | | |
| P15907 | Beta-galactoside alpha-2,6-sialyltransferase 1 | 675195 | | | |
| Q9Y376 | Calcium-binding protein 39 | 173360 | | | |
| O75874 | Isocitrate dehydrogenase [NADP] cytoplasmic | 461314 | | | |
| Q96LZ2 | Melanoma-associated antigen B10 | 281799 | | | |
| Q15155 | Nodal modulator 1 | 153276 | | | |
| Q5JPE7 | Nodal modulator 2 | 153276 | | | |
| P69849 | Nodal modulator 3 | 153276 | | | |
| Q9NRG1 | Phosphoribosyltransferase domain-containing protein 1 | 318375 | | | |
| 094915 | Protein furry homolog-like | 1488362 | | | |
| P35580 | Myosin-10 | 228701 | | | |
| Q7Z333 | Probable helicase senataxin | 1833287 | | | |
| Q96M60 | Protein FAM227B | 151558 | | | |
| P48594 | Serpin B4 | 608951 | | | |

Further, each protein was judged to have some effectiveness as a biomarker for the detection of the tau pathogenesis/neurodegenerative phase in cases where the ratio of the average amount of the protein in the extracellular vesicle fraction of Group 1 (A+T+N+ group) to the average amount of the protein in the extracellular vesicle fraction of Group 2 (A-T-N- group) was not less than 2 or not more than 0.5, or where the expression of the protein was found only in one of Group 1 and Group 2. As a result, the following molecular species were selected (Table 3): cholinesterase, pericentrin, protein unc-13 homolog C, 26S proteasome non-ATPase regulatory subunit 1, alpha-(1,3)-fucosyltransferase 10, neurexophilin-1, DNA-binding protein RFX5, pregnancy-specific beta-1-glycoprotein 8, amyloid-beta A4 precursor protein-binding family B member 2, kallikrein-11, cadherin-11,beta-galactoside alpha-2,6-sialyltransferase 1, sodium channel protein type 4 subunit alpha, protein FAM227B, and transmembrane protein 62. In addition, these molecular species may be useful as potential targets of therapeutic/prophylactic agents.

**Table 3 A + T + N - / A - T - N -**

| **Accession No.** | **Protein name** | **Abundances (T+[N+])** | **Abundances (T-[N-])** | **Ratio (T+[N+]/T-[N-])** | **p value(t test)** |
|---|---|---|---|---|---|
| P06276 | Cholinesterase | 954055 | 333900 | 3.35 | 0.0113 |
| O95613 | Pericentrin | 314273 | 125524 | 3.21 | 0.0138 |
| Q8NB66 | Protein unc-13 homolog C | 1983792 | 939780 | 2.11 | 0.0087 |
| Q99460 | 26S proteasome non-ATPase regulatory subunit 1 | 695942 | 1589799 | 0.44 | 0.0036 |
| Q6P4F1 | Alpha-(1,3)-fucosyltransferase 10 | 139241 | 351706 | 0.40 | 0.0232 |
| P58417 | Neurexophilin-1 | 219675 | 659634 | 0.33 | 0.0153 |
| P48382 | DNA-binding protein RFX5 | 98697 | 332078 | 0.30 | 0.0255 |
| Q9UQ74 | Pregnancy-specific beta-1-glycoprotein 8 | 407582 | | | |
| Q92870 | Amyloid-beta A4 precursor protein-binding family B member 2 | 97610 | | | |
| Q9U8X7 | Kallikrein-11 | 279781 | | | |
| P55287 | Cadherin-11 | | 120486 | | |
| P15907 | Beta-galactoside alpha-2,6-sialyltrarsferase 1 | | 676195 | | |
| P35499 | Sodium channel protein type 4 subunit alpha | | 136218 | | |
| Q96M60 | Protein FAM227B | | 151558 | | |
| Q0P6H9 | Transmembrane protein 62 | | 1364335 | | |

### (Example 2)

### Measurement of Cathepsin B

Among the molecular species selected as amyloid accumulation phase markers, cathepsin B was subjected to measurement of its concentration in extracellular vesicle fractions of cerebrospinal fluid specimens and plasma specimens, using ELISA.

### Details of Subjects

For the cathepsin B measurement, the following subject specimens were used (Table 4). From each subject specimen, a cerebrospinal fluid specimen and a plasma specimen were collected. Also for these subject specimens, all subjects had been classified into the normal group, MCI group, and AD group based on clinical diagnosis (data not shown). Of the cerebrospinal fluid specimens collected from the subject specimens, the cerebrospinal fluid specimens for Aβ1-42/p-Tau181/t-Tau ELISA described below were used to measure Aβ, phosphorylated tau, and total tau by ELISA. Based on their measured values, ROC analysis was performed between the normal + MCI group and the AD group based on clinical diagnosis of all subjects, to set cut-off values (Aβ: 747.2 pg/mL, phosphorylated tau: 58.4 pg/mL, and total tau: 756.6 pg/mL) for the ATN classification.

**Table 4**

| ATN classification | Number of specimen |
|---|---|
| A-T-N- | 36 |
| A-T+N- | 24 |
| A-T-N+ | 12 |
| A-T+N- | 11 |
| A+T-N- | 7 |
| A+T+N- | 16 |
| A+T+N+ | 23 |
| A+T-N+ | 7 |
| Total | 136 |

### Collection of cerebrospinal fluid, and method of collection of cerebrospinal fluid

Lumbar puncture was performed for subjects of 40 to 80 years old who were taking neither an antiplatelet drug nor anticoagulant, and who had no blood disease. The participants had finished dinner by 21:00 on the previous day, and slept for not less than 6 hours without taking alcohol. After overnight fasting, lumbar puncture was performed between 9:00 AM and 10:00 AM. Using a 22G top spinal needle with a steel needle and a polypropylene base, cerebrospinal fluid was collected. Thereafter, cerebrospinal fluid specimens for Aβ1-42/p-Tau181/t-Tau ELISA were collected by performing the following Process 1, and cerebrospinal fluid specimens for EV analysis were collected by performing the following Process 2. All specimens were collected by a single experienced physician by the same methods using the same apparatuses, at the same time of day.

### 1. Cerebrospinal fluid specimens for Aβ1-42/p-Tau181/t-Tau ELISA

The cerebrospinal fluid that had dropped from the spinal needle during the collection process was collected into a sterile 15-cc polypropylene tube. Thereafter, centrifugation was carried out at room temperature at 3500 rpm (2200 g) for 7 minutes to precipitate cells and other insoluble substances. Subsequently, using a polypropylene dispensing tip for ELISA, the supernatant was aliquoted in 500-µl volumes into polypropylene microtubes (1.5ml). Within one hour after the collection, these aliquots were cryopreserved at -80°C, and stored until the subsequent analysis. The freeze-thaw cycle was carried out only once.

### 2. Spinal Fluid Specimens for EV Analysis

Cerebrospinal fluid was directly collected dropwise from the spinal needle during the collection process into polypropylene microtubes (1.5 ml) in an amount of 500 µl/tube. Within one hour after the collection, the cerebrospinal fluid was cryopreserved at -80°C, and stored until the subsequent analysis. The freeze-thaw cycle was carried out only once.

### Collection of Plasma

After the cerebrospinal fluid was collected by the above method, whole blood was collected from the left upper arm of the same patient into a vacuum tube containing EDTA 2Na. Thereafter, the collected whole blood was centrifuged at 2200 g at 4°C for 15 minutes to collect plasma, and the collected plasma was aliquoted in about 0.5-ml volumes into sterile 15-cc polypropylene tubes. All samples were frozen at -80°C within one hour after the collection, and stored until the subsequent analysis. The freeze-thaw cycle was carried out only once.

### Extracellular Secreted Vesicle Fraction

From 0.1 mL of the cerebrospinal fluid specimen for EV analysis or 0.4 mL of the plasma specimen, an extracellular vesicle fraction was prepared. The preparation of the extracellular vesicle fraction was carried out using ultracentrifugation. In the ultracentrifugation, a series of operations (2000 g for 10 minutes, 10,000 g for 30 minutes, and then 100,000 g for 70 minutes) were repeated three times.

### ELISA for Cathepsin B

The extracellular vesicle fraction collected was dissolved in 0.1% SDS/PBS, and the concentration of cathepsin B was measured using a Human Cathepsin B ELISA Kit (Abcam: #ab119584) according to a recommended protocol.

### Results

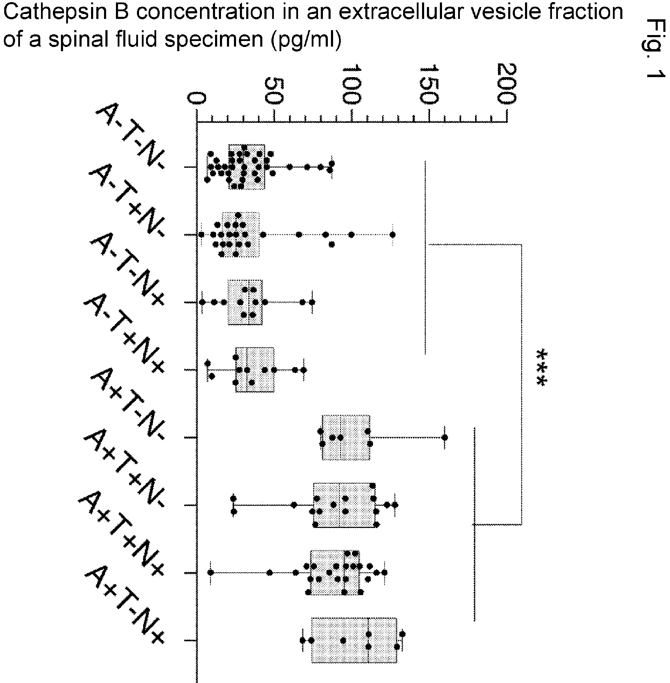

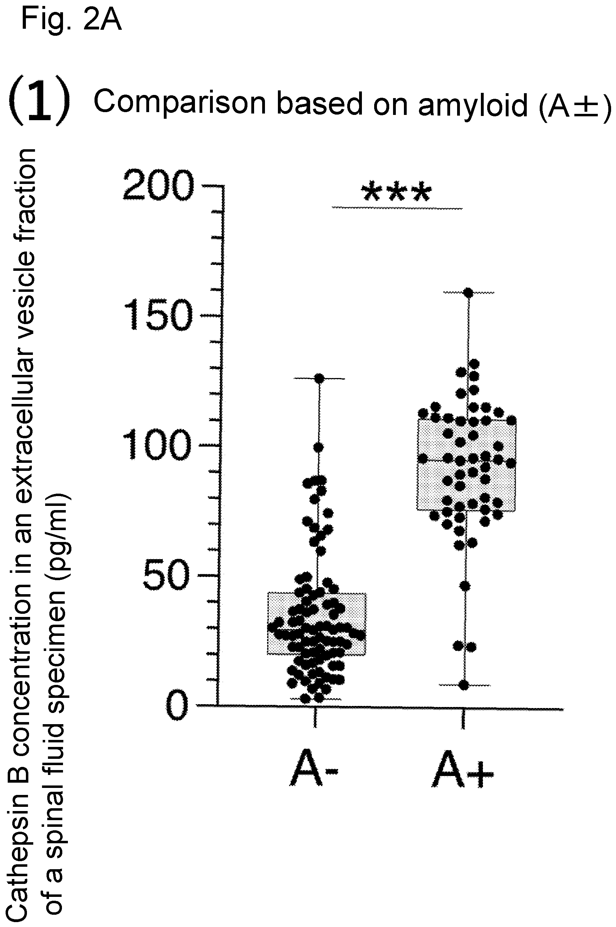

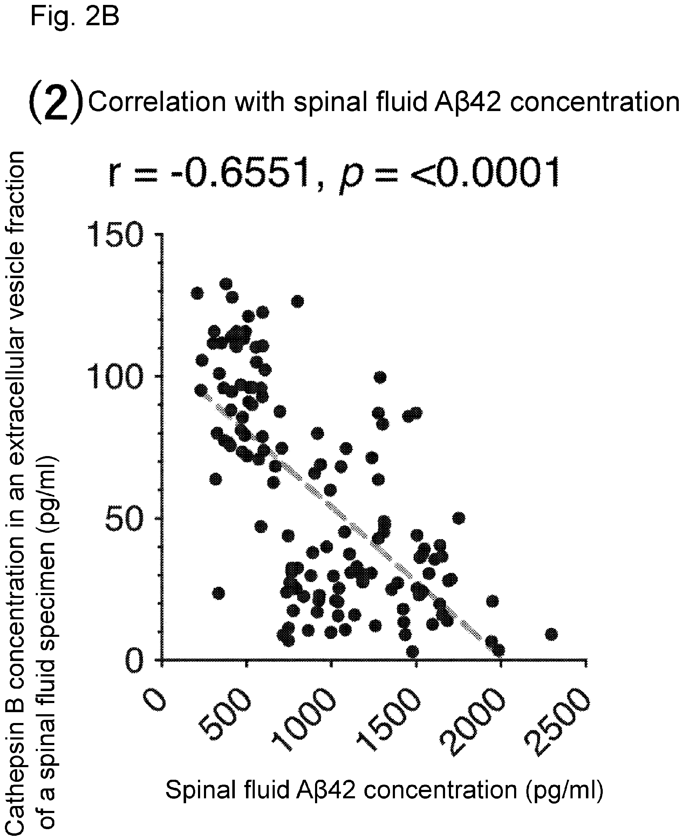

The results of the ELISA for cathepsin B in the extracellular vesicle fraction of the cerebrospinal fluid specimens are shown in Fig. 1 and Fig. 2.

From the results on the ATN classification and the concentration of cathepsin B shown in Fig. 1, it was found that the A+T-N- group has significantly increased concentrations of cathepsin B compared to the A-T-N- group, indicating that cathepsin B is effective as a biomarker for detecting the amyloid accumulation phase in accordance with the result on the cerebrospinal fluid extracellular vesicle proteomics described above. Further, the concentration of cathepsin B was significantly increased in each of the A+T+N+ group, A+T-N+ group, and A+T-N-group relative to any of the A-T+N+ group, A-T-N+ group, and A-T-N- group. This suggests that cathepsin B is useful also as a marker for detecting the tau pathogenesis/neurodegenerative phase.

Focusing on the amyloid pathology, the specimens were compared after classification into the A+ group (A+T+N+ group, A+T-N+ group, and A+T-N- group) and the A- group (A-T+N+ group, A-T-N+ group, and A-T-N- group) (Fig. 2A). As a result, it was found that the cathepsin B concentration is significantly increased in the A+ group, and that the cerebrospinal fluid Aβ42 concentration, which serves as an index of the amyloid pathology, is inversely correlated with the cathepsin B concentration in the extracellular vesicle fraction (Fig. 2B). Thus, cathepsin B was again found to be effective as a biomarker for detecting the amyloid accumulation phase.

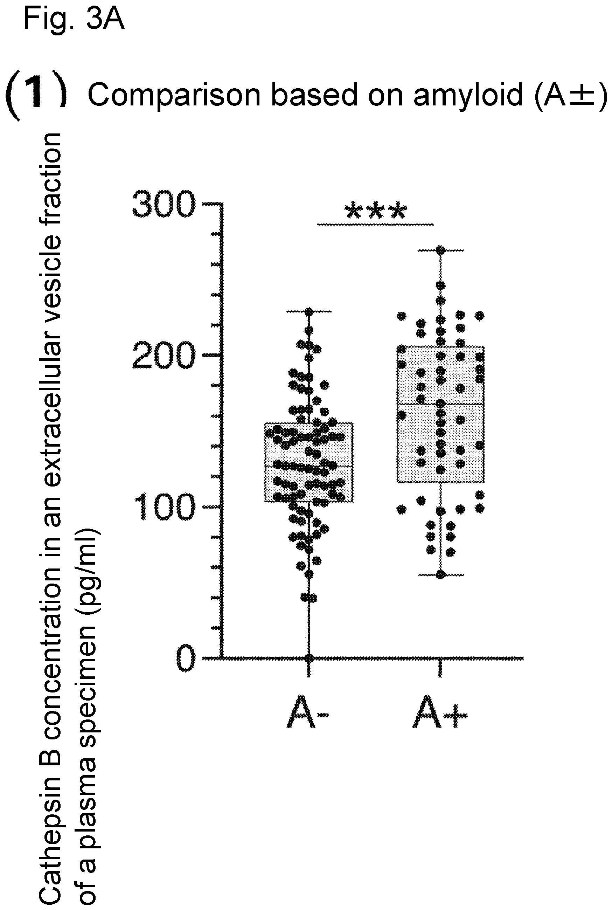

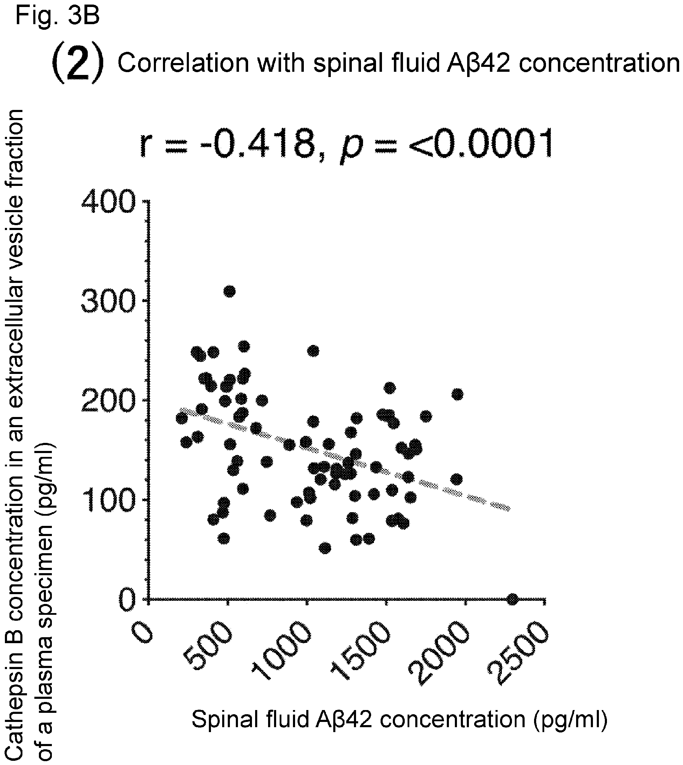

The results of the ELISA for cathepsin B in the extracellular vesicle fraction of the plasma specimens are shown in Fig. 3.

From the results shown in Fig. 3A, the plasma specimens, as well as the cerebrospinal fluid specimens, were found to have significantly higher cathepsin B concentrations in the A+ group than in the A- group. Further, as in the results for the cerebrospinal fluid specimens, the cathepsin B concentration in the extracellular vesicle fraction of the plasma specimens was found to be inversely correlated with the cerebrospinal fluid Aβ42 concentration (Fig. 3B).

These results indicate that cathepsin B is effective as a biomarker for detecting the amyloid accumulation phase even in cases where plasma is used as the specimen.

Thus, cathepsin B contained in the extracellular vesicle fraction was found to be an effective cerebrospinal fluid/blood biomarker for Alzheimer's disease, which biomarker is even capable of judging Alzheimer's disease in the preclinical phase/early phase including the amyloid accumulation phase.

## Claims

1. A method of detecting Alzheimer's disease, comprising the step of:
measuring the quantity of a protein present in extracellular vesicles in a sample collected from a specimen.

2. The method according to claim 1, wherein the amount of the protein contained in extracellular vesicles in an Alzheimer's disease specimen is different from an amount of the protein contained in extracellular vesicles in a normal specimen.

3. The method according to claim 2, wherein the difference is by a factor of not less than 2 or not more than 0.5.

4. The method according to any one of claims 1 to 3, comprising the step of:
collecting an extracellular vesicle fraction from the sample collected from the specimen, and measuring the protein contained in extracellular vesicles in the extracellular vesicle fraction.

5. The method according to any one of claims 1 to 4, wherein the protein contained in the extracellular vesicles is one or more proteins selected from the group consisting of: ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA 2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B10, Nodal modulator 1, Nodal modulator 2, Nodal modulator 3, phosphoribosyltransferase domain-containing protein 1, protein furry homolog-like, myosin-10, helicase senataxin, protein FAM227B, serpin B4, cholinesterase, pericentrin, protein unc-13 homolog C, 26S proteasome non-ATPase regulatory subunit 1, neurexophilin 1, DNA-binding protein RFX5, pregnancy-specific beta-1-glycoprotein 8, amyloid-beta A4 precursor protein-binding family B member 2, kallikrein-11, cadherin-11, sodium channel protein type 4 subunit alpha, and transmembrane protein 62.

6. The method according to any one of claims 1 to 5, wherein the protein contained in the extracellular vesicles includes cathepsin B.

7. A method of detecting an amyloid accumulation phase of Alzheimer's disease, the method comprising the step of:
measuring a protein contained in extracellular vesicles in a sample collected from a specimen.

8. The method according to claim 7, wherein the protein contained in the extracellular vesicles is one or more proteins selected from the group consisting of: ectonucleotide pyrophosphatase/phosphodiesterase family member 3, alpha-(1,3)-fucosyltransferase 10, repulsive guidance molecule A, cAMP-dependent protein kinase type I-beta regulatory subunit, DNA replication ATP-dependent helicase/nuclease DNA 2, contactin-6, histone H4, exostosin-1, BCL-6 corepressor-like protein 1, immunoglobulin mu heavy chain, Rho guanine nucleotide exchange factor 28, cathepsin B, putative keratin-87 protein, RNA-binding protein with multiple splicing, serpin B3, beta-galactoside alpha-2,6-sialyltransferase 1, calcium-binding protein 39, isocitrate dehydrogenase [NADP] cytoplasmic, melanoma-associated antibody B10, Nodal modulator 1, Nodal modulator 2, Nodal modulator 3, phosphoribosyltransferase domain-containing protein 1, protein furry homolog-like, myosin-10, helicase senataxin, protein FAM227B, and serpin B4.

9. The method according to claim 7 or 8, wherein the protein contained in the extracellular vesicles includes cathepsin B.

10. A method of detecting a Tau pathogenesis phase and/or a neurodegenerative phase of Alzheimer's disease, the method comprising the step of:
measuring a protein contained in extracellular vesicles in a sample collected from a specimen.

11. The method according to claim 10, wherein the protein contained in the extracellular vesicles is one or more proteins selected from the group consisting of: cholinesterase, pericentrin, protein unc-13 homolog C, 26S proteasome non-ATPase regulatory subunit 1, alpha-(1,3)-fucosyltransferase 10, neurexophilin 1, DNA-binding protein RFX5, pregnancy-specific beta-1-glycoprotein 8, amyloid-beta A4 precursor protein-binding family B member 2, kallikrein-11, cadherin-11, beta-galactoside alpha-2,6-sialyltransferase 1, sodium channel protein type 4 subunit alpha, protein FAM227B, and transmembrane protein 62.

12. The method according to claim 10 or 11, wherein the protein contained in the extracellular vesicles includes cathepsin B.

13. The method according to any one of claims 1 to 12, wherein the sample is cerebrospinal fluid or plasma.

14. A diagnostic kit for Alzheimer's disease, comprising a reagent for measuring a protein contained in extracellular vesicles.
